# EUROPÄISCHE PATENTANMELDUNG

(11) **EP 2 410 042 A1**
(43) Veröffentlichungstag der Anmeldung: **25.01.2012**
(21) Anmeldenummer: 11005861.7
(22) Anmeldetag: 18.07.2011
(51) Int. Cl.: C12M 1/00

(54) **Bioreaktor zur Produktion von Miroorganismen**

(30) Priorität: 23.07.2010 DE 102010032108
(71) Anmelder: Forschungszentrum Jülich Gmbh, 52425 Jülich (DE)
(72) Erfinder: Jung, Hans, 52134 Herzogenrath (DE); Brehm, Thorsten, 52445 Titz (DE); Egmen, Ayhan, 52499 Baesweiler (DE)

(57) **Zusammenfassung**

Die erfindungsgemäße Vorrichtung umfasst einen transparenten Folienschlauch, der mit einer Verschlussanordnung verschlossen wird. Diese Verschlussanordnung setzt sich zusammen aus einer Verschlussplatte mit eingearbeiteten Anschlüssen und einem Verschlussring, der mit der unteren Verschlussplatte verschraubt oder verbunden wird.

## Beschreibung

Die Erfindung betrifft Bioreaktoren, im Weiteren auch Vorrichtungen genannt, für biotechnologische Produktions- und Verarbeitungsprozesse von Mikroorganismen.

Mit Hilfe von Bioreaktoren können sowohl nicht-phototrophe als auch phototrophe Mikroorganismen, wie beispielsweise Algen, Cyanobakterien oder Purpurbakterien kultiviert werden. In diesen Reaktoren wird entweder das Wachstum und die Vermehrung dieser Zellen ermöglicht oder die Produktion unterschiedlicher Substanzen mittels dieser Zellen. Solche Bioreaktoren sind in der Literatur ausreichend beschrieben.

Ein wichtiges Einsatzgebiet von beispielsweise Photobioreaktoren ist die Erzeugung von Algen, die zur Primärproduktion von Biomasse eine Rolle spielen. Algen sind wichtige CO₂ Konsumenten und können deshalb umweltentlastend wirken. Zu den Algen zählen einerseits die prokaryotischen Cyanobakterien als auch die eukaryotischen mikroskopischen Algenklassen. Diese Organismen liefern eine Vielfalt von Substanzklassen, die sich für Zwecke der Pharmazie, Kosmetik, Nahrung, Tierernährung sowie für technische Zwecke einsetzen lassen.

Als Bioreaktoren sind beispielsweise Platten-, Rohr oder Blasensäulenreaktoren oder auch Folienreaktoren bekannt.

Folienreaktoren werden in der Biotechnologie zur Produktion pflanzlicher Rohstoffe, zur Zucht von photoautotrophen und nicht-phototrophen Mikroorganismen wie Bakterien, Hefearten und Hefepilzen, zur Aufzucht von Zooplankton, zur Umwandlung bzw. Verwertung von Nitraten, Phosphaten und CO₂ im Rahmen eines Wachstumsprozesses eingesetzt. Folienreaktoren sind grundsätzlich für Anwendungen einsetzbar, wo Partikel biologischer oder nicht biologischer Herkunft in gleichzeitigen Kontakt mit Gas, rein oder als Mischgas, und/oder Luft, und/oder Licht (Kunst- oder natürliches Licht) und/oder Flüssigkeit gebracht werden.

Die nach dem Stand der Technik bekannten Folienreaktoren (z. B. DE 20 2004 002 536U1) bestehen aus transparenten Kunststoffschläuchen, die in V-Form an ein tragendes Rohrgerüst befestigt sind. Im unteren Bereich des wassergefüllten V-Systems werden Kunststoffkanülen eingeführt, die als Ausströmer für Druckluft und CO₂ dienen und weitere Kanülen, die zur Zufuhr von Nährstoffen und Frischwasser oder zur Ernte der Mikroalgen verwendet werden können.

### Nachteile des Stands der Technik:

Die bisher bekannten Folienreaktoren weisen den Nachteil auf, dass nur Zugangsschläuche mit geringem Durchmesser verwendet werden können. Dadurch treten Verstopfungen in den Zugangsschläuchen auf, was zu einer verringerten Umwälzung des Mediums führen kann. Ein weiterer Nachteil der in die Folienreaktoren gesteckten Zugangsschläuche ist die Gefahr des Herausrutschens und Auftretens von Undichtigkeiten. Im unteren Knick des V-förmigen Reaktors lagert sich mit zunehmender Betriebsdauer des Reaktors vermehrt Biomasse an. Dies führt zu Fäulnisprozessen und unerwünschtem Bakterienwachstum. Weiterhin führt die zunehmende Biomasse zu Verstopfungen des Zugangs zwischen der rechten und linken Reaktorseite. Ein gleichmäßiger Austausch im Reaktor ist dadurch nicht mehr gegeben, so dass sich unterschiedliche Medienbedingungen, wie z. B. unterschiedliche pH-Werte einstellen können.

### Aufgabe der Erfindung:

Es ist daher Aufgabe der Erfindung, einen Folienreaktor zu schaffen, bei dem eine Durchmischung des Reaktors verbessert und die Entstehung von Toträumen verringert wird, so dass das Auftreten von Verstopfungen vermindert wird und weiterhin eine verbesserte Befestigungsmöglichkeit für Zu- und Ablaufleitungen möglich wird. Dadurch soll weiterhin der Betrieb des Folienreaktors wartungsärmer werden und stabile Betriebsbedingungen (wie z. B. gleichmäßige Durchmischung im Reaktor, stabile/r pH-Wert, Temperatur, Leitfähigkeit, Nährstoffkonzentration) geschaffen werden.

### Lösung der Probleme durch die Erfindung:

Die erfindungsgemäße Vorrichtung umfasst einen transparenten Folienschlauch, der mit einer Vorschlussanordnung verschlossen wird. Diese Verschlussanordnung setzt sich zusammen aus einer Verschlussplatte mit eingearbeiteten Anschlüssen und einem Verschlussring, der mit der unteren Verschlussplatte verschraubt oder verbunden wird.

Durch das Verschließen des Folienschlauchs mit der erfindungsgemäßen Verschlussanordnung, in die bereits Anschlüsse für die Zu- und Abfuhr von Flüssigkeiten und Gasen eingearbeitet sind (z. B. für Luftzufuhr, CO₂-Zufuhr, Zulauf- und Ablaufanschlüsse), werden Leckagen, die zuvor durch das Einstechen von Kanülen in die Folienschläuche auftraten, verhindert.

Durch die erfindungsgemäße Ausgestaltung der Vorrichtung mit der Verschlussanordnung wird ein durchgehend rohrförmiger Querschnitt der Vorrichtung erreicht. Dieser rohrförmige Querschnitt führt dazu, dass kein Bereich des Folienschlauches abgeknickt wird. Eine Ablagerung von Biomasse in diesem Bereich wird dadurch verhindert.

Durch die erfindungsgemäße Ausgestaltung mit den Anschlüssen in der Verschlussplatte müssen in den Folienschlauch keine Kanülen gestochen werden, so dass der Folienschlauch mehrfach verwendet werden kann. Die Anzahl der Anschlüsse kann an den Bedarf des Produktions- und Verarbeitungsprozesses angepasst werden. So können beispielsweise 4, 6 oder auch 8 Anschlüsse in die Verschlussplatte eingelassen sein.

Die Vorrichtung kann als einzelne Foliensäule unter Luftdruck wie ein stehendes Rohr oder auch hängend mit nach dem Stand der Technik bekannten Haltevorrichtungen genutzt werden.

Die Verschlussplatte umfasst einen zylindrischen Abschnitt mit einem Ringbund. Der Verschlussring steht über die Außenmantelfläche und die Bundfläche mit der Verschlussplatte in Verbindung. Durch den Anpressdruck des Verschlussrings auf die Verschhissplatte wird das Abdichten der Vorrichtung erreicht.

In einer vorteilhaften Ausgestaltung der Vorrichtung weist die Verscblussplatte eine Nut auf. Diese dient zur Aufnahme des Folienschlauchs. Der Folienschlauch kann über die Verschlussplatte bis zum Nutgrund gestülpt werden und kann ohne zu Verrutschen mit der unteren Verschlussplatte verschlossen werden. Je tiefer bzw. länger die Nut ausgestaltet ist, desto größer wird die Fläche; die in Verbindung steht mit dem Folienschlauch und der Verschlussplatte. Je tiefer die Nut ausgestaltet ist, desto besser steht der Folienschlauch daher mit der Verschlussanordnung in Kontakt und führt zu einem Leckage freien Verschließen der Vorrichtung. Beispielhaft kann die Tiefe der Nut zwischen ca. 10 bis 15 mm liegen. Die Nutbreite ist abhängig von der Dicke der verwendeten Folie. Die Nutbreite sollte mindestens die Dicke der Folie aufweisen und, kann beispielsweise im Bereich von ca.1- 2 mm liegen.

In einer weiteren vorteilhaften Ausgestaltung der erfindungsgemäßen Vorrichtung weist der Verschlussring eine abgeschrägte Kante auf, die über einen Dichtring den Folienschlauch an die Verschlussplatte drückt. Durch die Schräge wird die Fläche, die auf den Dichtring drückt, vergrößert und damit eine verbesserte Kraftübertragung auf die Wichtung bzw. den Dichtring beim Verschrauben des Verschlussrings mit der Verschlussplatte erreicht. Durch die abgeschrägte Kante wird die Kraft, die durch das Verschrauben des Verschlussrings mit der Verschlussplatte ausgeübt wird, so umgelenkt und verteilt, dass sie nicht nur in Richtung der Verschlussplatte gerichtet ist, sondern auch auf die volle radiale Erstreckung der umlaufenden Bundfläche der Verschlussplatte gerichtet ist. Der Folienschlauch kann dadurch so zwischen Verschlussring und Verschlussplatte fest gepresst werden, dass die Vorrichtung auslaufsicher abgedichtet werden kann.

Die Schräge kann beispielsweise eine Neigung von 30° bis 60° haben. In einer besonders vorteilhaften Ausgestaltung der Vorrichtung kann die Schräge eine Neigung von 45° aufweisen.

Die Verschlussanordnung kann bevorzugt einen kreisfomugen bis ellipsoiden Querschnitt ausweisen. Weiterhin möglich ist aber auch ein polygonaler Querschnitt.

Durch Verbinden mehrerer Folienschläuche mit einem Verbindungsrohr, welches jeweils mit der unteren Verschlussplatte einer weiteren Verschlussanordnung verbunden ist, können mehrere Folienschläuche über eine Reihenanordnung zu einem großen System beliebig erweitert werden. Das Verbindungsrohr kann beispielsweise U-förmig ausgestaltet sein.

Als Materialen für die Verschlussanordnung sind alle nach dem Stand der Technik in der Produktion von Mikroorganismen verwendeten Materialien geeignet, wie z. B. Kunststoffe, Metalle oder Glas.

Zunächst wird die Folie über den Ringbund der unteren Verschlussplatte bis in den Nutgrund gestülpt. Dann wird ein Dichtring, z. B. O-Ring, über den Folienschlauch gezogen. Anschließend wird der Verschlussring um den Folienschlauch herum gelegt und mit der unteren Verschlussplatte über Befestigungsmittel, z. B. Schrauben, Verschlussklammern, verbunden. Durch den Druck des Verschlussrings auf den Dichtring wird das Verrutschen des Folienschlauchs von der Verschlussplatte und ein Herauslaufen von Flüssigkeit verhindert. In einer vorteilhaften Ausgestaltung der Vorrichtung entspricht der Außendurchmesser des Ringbundes dem Innerdurchmesser des Folienschlauchs. So wird gewährleistet, dass eine passgenaue abdichtung des Folienschlauchs mit der Verschlussanordnung möglich ist. Beispielhaft kann der Durchmesser des Ringbundes in einem Bereich von ca. 160 bis 180 mm liegen.

### Spezieller Beschseibungsteil

Nachfolgend wird der Gegenstand der Erfindung anhand von Figuren näher erläutert ohne dass der Gegenstand der Erfindung Dadurch beschränkt wird. Es ist gezeigt:
- Fig 1:: Ausschnitt der erfindungsgemäßen Vorrichtung mit Verschlussanordnung
- Fig 2:: Vorrichtung mit Verschlussanordnung im Querschnitt gemäß Schnittebene A aus Fig. 1
- Fig 3:: Vergrößerte Darstellung der Verschlussanordnung im Bereich B
- Fig 4:: Zwei erfindungsgemäße Vorrichtungen, die miteinander über die Verschlussanordnung verbunden sind

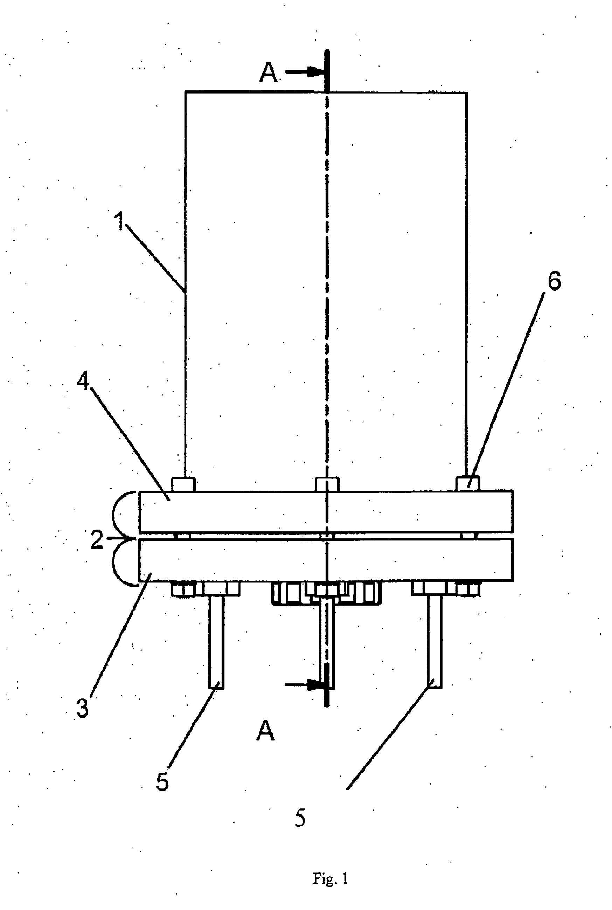

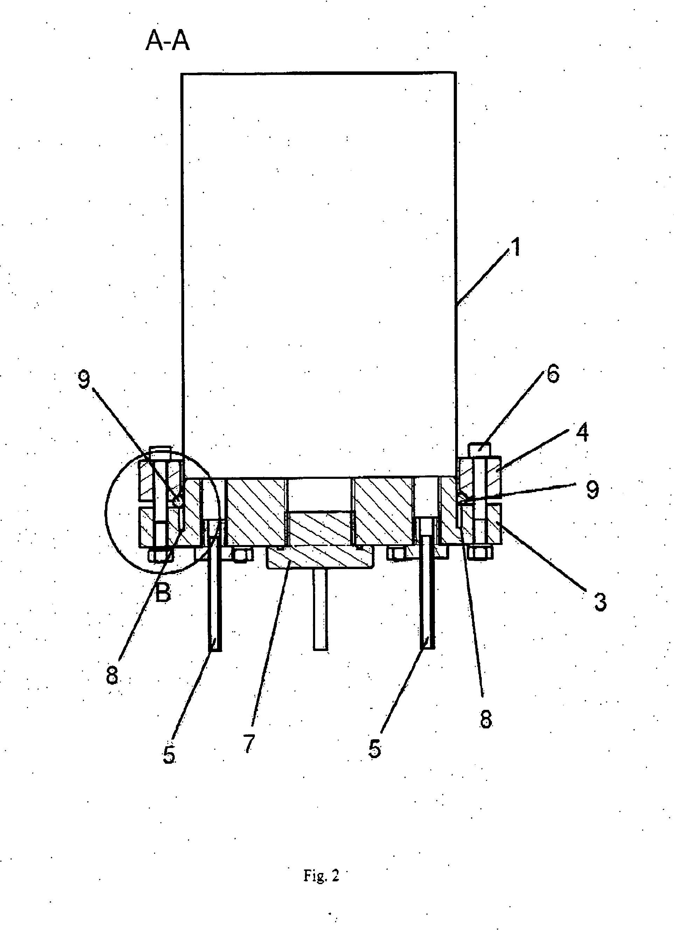

Figur 1 zeigt ein Ausführungsbeispiel der erfindungsgemäßen Vorrichtung. Diese umfaßt den transparenten Folienschlauch (1) sowie die Verschlussanordnung (2), die sich zusammensetzt aus einer unteren Verschlussplatte (3) und einem oberen Verschlussring (4). Weiterhin sind an der unteren Verschlussplatte (3) Anschlüsse (5) für Zu- und Abfuhr von Flüssigkeiten oder Gasen angeordnet. Der Verschlussring (4) wird über Befestigungsmittel (6) mit der Verschlussplatte (3) verbunden. Die gestrichelte vertikale Linie "A" gibt die Schnittebene an, die in Figur 2 dargestellt wird.

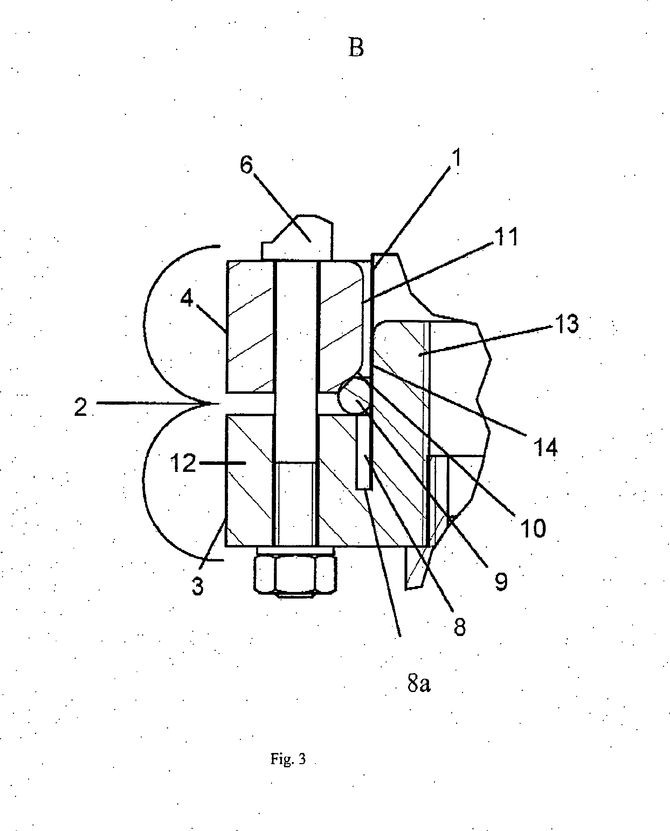

Figur 2 zeigt die Vorrichtung gemäß Schnittebene A aus Figur 1. Die Verschlussplatte (3) umfasst hier einen verschraubbaren Stopfen (7) mit dem ein Zugang/ Ausgang zum Folienschlauch (1) geschaffen wird. In die Verschlussplatte (3) sind weiterhin Anschlüsse (5) für die Zufuhr/Abfuhr von Flüssigkeiten eingelassen. Der Verschlussring (4) ist über Befestigungsmittel (6) mit der Verschlussplatte (3) verbunden. Die Verschlussplatte (3) weist darüber hinaus eine radial umlaufende Nut (8) auf, in die der Folienschlauch (1) eingelassen ist. Der Folienschlauch wird über einen Dichtring (9) mit Hilfe des Verschlussrings (4) an die Verschlussplatte (3) gepresst, so dass ein dichter Abschluss des Folienschlauches (1) mit der Verschlussanordnung (2) erreicht wird. Der im unteren linken Figurbereich mit "B" gekennzeichnete Kreis markiert den in Figur 3 vergrößert dargestellten Ausschnitt.

Figur 3 zeigt einen vergrößert dargestellten Ausschnitt der Figur 2 im Bereich "B". Der Verschlussring (4) weist eine abgeschrägte Kante (10) auf, die sich an der axial zur Verschlussplatte (3) weisenden Innenfläche (11) des Verschlussrings (4) befindet und an den Dichtring (9) grenzt. Der Folienschlauch (1) ist in die Nut (8) bis zum Nutgrund (8a) hineingeschoben. Die Verschlussplatte (3) weist, einen zylindrische Abschnitt (12) sowie einen Ringbund (13) auf. Der Verschlussring (4) drückt mit seiner axialweisenden Innenfläche (11) über den Dichtring (9) vorwiegend an die Bundfläche (14) der Verschlussplatte (3). Dadurch können der Verschlussring (4) und der Ringbund (13) der Verschlussplatte (3) auf der vollen radialen Erstreckung der umlaufenden Bundfläche (14) kontaktieren und über den Dichtring (9) im Bereich des Außendurchmessers des Ringbundes (13), der Verschlussplatte (3) Kräfte übertragen und so den Folienschlauch (1) fest und dicht mit der Verschlussanordnung (2) verschließen. Weiterhin drückt der Verschlussring (4) auf den zylindrischen Abschnitt (12) der Verschlussplatte (3), so dass der Folienschlauch (1) nicht herausrutschen kann oder Leckagen entstehen.

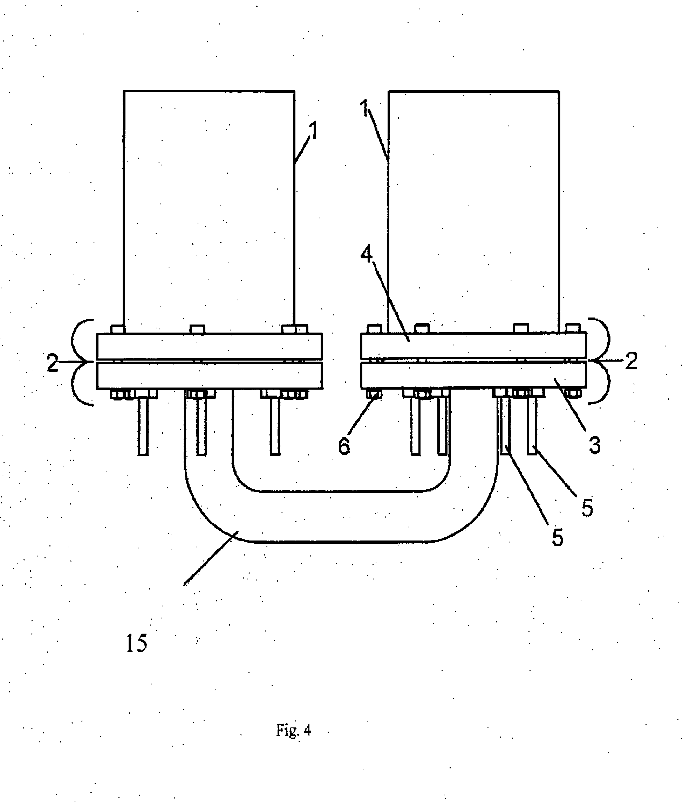

Figur 4 zeigt eine Ausführung der Vorrichtung, bei der zwei Folienschläuche (1) miteinander kombiniert und verbunden sind. Dabei sind die Verschlussanordnungen (2) und damit auch die Folienschläuche (1) durch ein Verbindungsrohr (15) miteinander verbunden. Das Verbindungsrohr (15) ist über die Stelle, an der zuvor der verschraubbare Stopfen (7) eingelassen war, so an die untere Verschlussplatte (3) angeschlossen, dass ein Zugang/Durchgang zum Folienschlauch (1) besteht.

## Patentansprüche

1. Vorrichtung zur Produktion von Mikroorganismen, umfassend einen transparenten Folienschlauch (1),
**dadurch gekennzeichnet, dass**
der Folienschlauch (1) mit einer Verschlussanordnung (2) verschlossen wird, der aus einer Verschlussplatte (3) mit eingearbeiteten Anschlüssen (5) und einem Verschlussring (4) besteht, wobei der Verschlussring (4) mit der unteren Verschlussplatte (3) über Befestigungsmittel (6) verbunden ist.

2. Vorrichtung nach Anspruch 1,
**dadurch gekennzeichnet, dass**
die Verschlussplatte (3) eine Nut (8) zur Aufnahme des Folieschlauchs (1) aufweist.

3. Vorrichtung nach einem der Ansprüche 1 bis 2,
**dadurch gekennzeichnet, dass**
ein Dichtring (9) zwischen Folienschlauch (1) und Verschlussring (4) angeordnet ist.

4. Vorrichtung nach einem der Ansprüche 1 bis 3,
**dadurch gekennzeichnet, dass**
der Verschlussring (4) an der axial zur Verschlussplatte (3) weisenden Innenfläche (11) eine abgeschrägte Kante (10) ausweist.

5. Vorrichtung nach vorhergehendem Anspruch 4,
**dadurch gekennzeichnet, dass** die abgeschrägte Kante (10) eine Neigung von 30 bis 60° aufweist.

6. Vorrichtung nach einem der Ansprüche 1 bis 5,
**dadurch gekennzeichnet,**
**dass** die Verschlussplatte (3) einen Ringbund (13) aufweist, dessen Außendurchmesser dem Innendurchmesser des Folienschlauchs (1) entspricht.

7. Vorrichtung nach einem der Ansprüche 1 bis 6,
**dadurch gekennzeichnet,**
**dass** einzelne Folienschläuche (1) über Verbindungsrohre (15) zu einem großen System miteinander verbunden sind.
